# EUROPEAN PATENT SPECIFICATION

(11) **EP 3 238 766 B1**
(45) Date of publication and mention of the grant of the patent: **05.12.2018**
(21) Application number: 15871516.9
(22) Date of filing: 08.04.2015
(51) Int. Cl.: A61M 16/00, A61M 16/08, G01M 3/26

(54) **AIRTIGHTNESS-DETECTING INTERMEDIATE PIPE ASSEMBLY**
ZWISCHENROHRANORDNUNG ZUR ERKENNUNG VON LUFTDICHTHEIT
ENSEMBLE TUYAU INTERMÉDIAIRE DE DÉTECTION D'ÉTANCHÉITÉ À L'AIR

(30) Priority: 23.12.2014 CN 201420826657 U
(43) Date of publication of application: 01.11.2017
(73) Proprietor: Shenzhen Envisen Industry Co., Limited, Shenzhen, Guangdong 518115 (CN)
(72) Inventor: LU, Yufei, Shenzhen, Guangdong 518115 (CN)
(74) Representative: McIlroy, Steven David
(86) International application number: PCT/CN2015/076021
(87) International publication number: WO 2016/101438

(56) References cited:
- EP-A1- 0 510 369
- CN-U- 201 586 307
- CN-U- 201 586 307
- CN-U- 202 777 348
- CN-U- 203 852 686
- CN-U- 203 852 686
- US-A- 5 121 746
- US-A1- 2014 150 794

## Description

### Technical field

The present invention relates to the field of medical equipment technology, and more specifically to an airtightness-detecting bi-lumen breathing circuit septum assembly.

### Prior art

Mechanical ventilation equipment is an important form of medical equipment, which supplies patients with the oxygen they require. Mechanical ventilation equipment usually consists of a breathing mask, a medical ventilator (comprising parts such as an oxygen supply machine and an anaesthetic machine) and a bi-lumen breathing circuit which connects the breathing mask and ventilator of the type disclosed in the applicant's prior Utility Model No. CN 203 852 686U. The sufficient airtightness of the bi-lumen breathing circuit as a channel for conveying gas is the determinant of effective gas transport when the ventilator is in use. The inner cavity of the bi-lumen breathing circuit contains a septum which partitions the circuit into two channels for transporting different types of gases. Gas leaking from the septum leads to the mixing of different gases, which may endanger the health of the patient given the different reactions that can result from the transport of different gases. It is therefore necessary to conduct an airtightness test for the septum inside the bi-lumen breathing circuit. There is, however, no way to detect the airtightness of the septum in any existing bi-lumen breathing circuits, presenting a safety risk to the users.

### Existing technical problems

The aim of the present invention is to overcome the shortcomings of the prior art described above, and to provide an airtightness-detecting bi-lumen breathing circuit septum assembly which can detect the airtightness of the circuit wall and septum, thereby ensuring safety.

### Solution to the existing problem

The present invention is realised by the following means: An bi-lumen breathing circuit septum assembly, comprising: a first joint for connecting a breathing mask and a second joint for connecting a ventilator, with said first joint and said second joint being connected by a bi-lumen breathing circuit which is a bendable circuit body used to transport gases, inside the cavity of which is contained a septum which partitions said bi-lumen breathing circuit into a first and second channel which are mutually isolated, wherein the assembly is a bi-lumen breathing circuit septum assembly further comprising: a partition plate for partitioning the cavity of said first joint into two mutually isolated sections; and a connection structure located at the partition plate for its connection with said septum, with one end of said septum being connected to said connection structure; and a testing stopper insertable into said first joint to block either the first or second channel.

Specifically, said connection structure is a recessed groove located on said partition plate, with the said septum being slotted into said recessed groove.

Specifically, the one end on which each of the said first and second joints connect to said circuit body is the connected end, with said connected end being inserted into the circuit body, and with said partition plate being located within the cavity of the said connected end.

Specifically, said partition plate partitions the inner cavity of the said connected end into two equal sections.

Specifically, said connected end is equipped with grooves for the installation of sealing rings.

Specifically, the connecting point of said first joint with said circuit body and that of said second joint with said circuit body are each sealed and protected with an airtight plastic layer. Said airtight plastic layer is wrapped around the corresponding surfaces of the said sealing rings.

Preferably, said circuit body is a corrugated pipe.

### Beneficial effects of the present invention

The present invention provides an airtightness-detecting bi-lumen breathing circuit septum assembly which contains, in the inner cavity of the first joint, a partition plate with a connection structure. In this way, the connection structures on the septum and partition plate are connected, therefore partitioning the circuit body into two mutually isolated channels. The leak test can then therefore be conducted on said septum when one of the said partitioned channels are blocked, thereby eliminating the risk involved in using traditional bi-lumen breathing circuits arising from the inability to detect leakage of the septum therein. The invention therefore makes it safer to use bi-lumen breathing circuits.

### Brief description of the drawings

Figure 1 is a schematic diagram showing the airtightness-detecting bi-lumen breathing circuit septum assembly provided in an embodiment of the present invention.
Figure 2 is a diagram showing the first joint of the airtightness-detecting bi-lumen breathing circuit septum assembly provided in an embodiment of the present invention.

### Embodiments of the invention

To further illustrate the aim of the present invention, its technical solution and advantages, the drawings will be referred to in conjunction with the embodiments hereafter to give a detailed description of the present invention. It should be appreciated that the embodiments of the present invention are merely provided for the purposes of illustration, with the present invention not being limited to the specific embodiments described below.

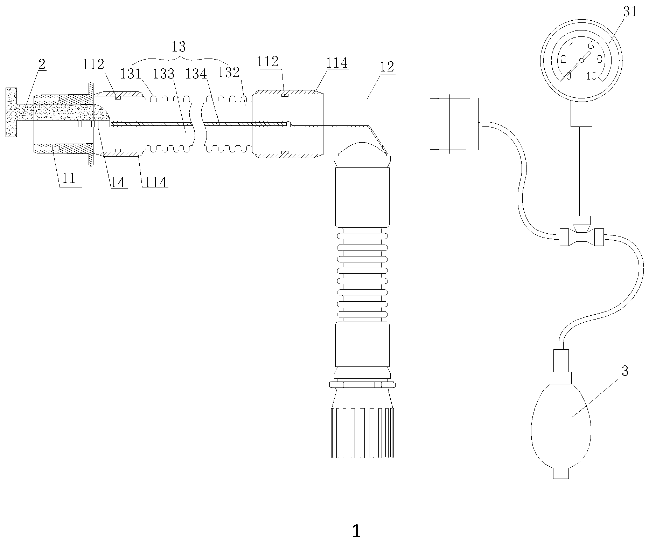

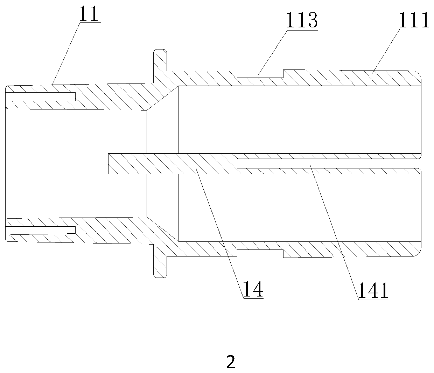

As indicated in Figures 1 and 2, the present invention offers an airtightness-detecting bi-lumen breathing circuit septum assembly, comprising: a first joint (11) for connecting a breathing mask, and a second joint (12) for connecting a ventilator, with said first joint (11) and said second joint (12) being connected by a bi-lumen breathing circuit (13) which comprises a bendable circuit body (131) for transporting gases, inside the cavity of which is contained a septum (134) which partitions the said bi-lumen breathing circuit body (131) into a first (132) and second channel (133) which are mutually isolated, a partition plate (14) to partition the cavity of said first joint (11) into two mutually isolated sections, and a connection structure (141) provided at the partition plate (14) for its connection with said septum (134), with one end of said septum (134) being connected to said connection structure (141). Such a design allows, in the course of manufacture and assembly, one end of the first joint (11) to be inserted into the circuit body (131), connecting one end of the septum (134) with the connection structure (141). Accordingly, the partition plate (14) and septum (134) together separate the whole circuit body (131) into mutually isolated first (132) and second (133) channels for the transport of different gases. The gases transported through the first (132) and the second (133) channels cannot be mixed, and it is therefore crucial that the septum (134) is airtight, in order to ensure the reliable transport of gases. Furthermore, in the present embodiment, the insertion of a partition plate (14) and the connection structure (141) which is used for connection with the septum (134) within the first joint (11) allows the complete mutual isolation of the first (132) and second channels (133) when the septum (134) and partition plate (14) are connected. When a test is to be conducted, after blocking the first joint (11), and completing the leak test for the circuit body (131), the airtightness of the septum (134) can be detected. The testing stopper (2) can then be inserted into the first joint (11) to block either the first (132) or second channel (133) by contact with the upper and lower hard walls, after which the second joint (12) can be connected with a ventilator or anaesthetic machine (3). The ventilator or anaesthetic machine (3) is then used to draw gas of a certain pressure into the channel over a continuous period of time. If the reading of the pressure gauge (31) on the ventilator or anaesthetic machine (3) does not change, the septum (134) is shown to have no leaking and is thus airtight. If the reading of the pressure gauge (31) changes, the septum (134) is then shown to not be airtight, and therefore unusable. In this manner, a test can be conducted on the airtightness of the septum (134), while a septum (134) which causes leaking may then be screened out to ensure safety when using bi-lumen breathing circuits.

This embodiment of the present inventionprovides an airtight-detecting bi-lumen breathing circuit septum assembly which contains in the inner cavity of the first joint (11) a partition plate (14) with a connection structure (141) on said partition plate (14) which connects said connection structure (141) with one end of the septum (134) in order to allow the septum (134) to partition the inner cavity of the circuit body (131) into two mutually isolated channels. In this case, by blocking one of the channels and allowing gases to enter from the other end, a test can be conducted on the airtightness of the septum (134), screening out bi-lumen breathing circuits containing septum (134) with leakages, preventing said bi-lumen breathing circuits (13) with leaking septums (134) from being used, thereby providing for safe use.

Specifically, as shown in Figures 1 and 2, the connection structure (141) is a recessed groove set on the partition plate (14), with the orientation of said recessed groove being the same as that of the long side of the partition plate (14), and said septum (134) being slotted into said recessed groove. The mode of connection between the septum (134) and the recessed groove is referred to as interference fit. Additionally, as the septum (134) is slotted into the recessed groove, a sealed plastic layer is laid between the two, ensuring their connectedness and that the point of connection remains airtight and firmly sealed.

Specifically, as shown in Figures 1 and 2, the respective ends connecting the circuit body (131) with the first (11) and second (12) joints are referred to as the connected end (111), with the other end of the first joint (11) being connected to the breathing mask. The connected end (111) is inserted into the circuit body (131), and the septum (14) located within the cavity of the connected end (111). Moreover, the septum (14) partitions the connected end (111) into two equal sections. In this case, the septum (14) partitions the connected end (111) into two equal sections to achieve the required specification for gas transport.

Specifically, as shown in Figures 1 and 2, to ensure the airtightness of the connection and to avoid leaking between the bi-lumen breathing circuit (13) and the first joint (11), a sealing groove (113) for the installation of a sealing ring (112) is located at the connection end (111). A sealing ring (112) is fitted on the sealing groove (113), with airtightness being further ensured by the connection end (111) fitting into the circuit body (131) with the sealing ring (112) properly fitted.

Specifically, as shown in Figures 1 and 2, the airtightness of the connection end may be subject to impact created by external forces under certain circumstances when it is in use, including when connected to the breathing mask and the ventilator, or when it does not remain stationery when in use. To ensure the airtightness of the connecting points and to avoid leakages, the connecting point between said first joint (11) and said circuit body (131) and that of said second joint (12) with said circuit body (131) are each sealed and protected with an airtight plastic layer (114). Said airtight plastic layer (114) is wrapped around the corresponding surfaces of said sealing rings (112). This configuration allows sealing to first be carried out with the sealing ring (112), then by inserting rubber or soft plastic by means of injection moulding or vulcanisation in the gaps of the connection ends, thus filling up the gap between the first joint (11) and the circuit body (131) and the gap between the second joint (12) and the circuit body (131) and welding the two parts into one and adhering to the sealing ring (112), forming into a reinforced airtight plastic layer (114), the reliability and strength of the airtightness of the connecting points can be ensured. A certain degree of external force will then be made tolerable so that the reinforced connection does not loosen, thus ensuring the reliable transport of gases.

Preferably, the circuit body (131) is a corrugated pipe, as its concertinaed surface allows for bending and deformation without damage.

The above description gives only the preferred embodiments of the present invention and is not used to limit the scope of protection thereof. All the modifications, equivalent replacements or improvements in the scope of the present inventionshall be included in the scope of protection of the present invention.

## Claims

1. A bi-lumen breathing circuit septum assembly, comprising: a first joint (11) for connecting a breathing mask and a second joint (12) for connecting a ventilator, with said first joint and said second joint being connected by a bi-lumen breathing circuit (13) which is a bendable circuit body (131) used to transport gases, inside the cavity of which is contained a septum (134) which partitions said bi-lumen breathing circuit (13) into a first and second channel (132, 133) which are mutually isolated, further comprising: a partition plate (14) for partitioning the cavity of said first joint (11) into two mutually isolated sections; and a connection structure (114) located at the partition plate (14) for its connection with said septum (134), with one end of said septum (134) being connected to said connection structure (114); **characterised in that**
the assembly further comprises a testing stopper (2) insertable into said first joint (11) to block either the first or second channel (132, 133) to allow detection of airtightness of the bi-lumen circuit septum.

2. The bi-lumen breathing circuit septum assembly according to claim 1, **characterised by** said connection structure (114) being a recessed groove located on said partition plate (14), with said septum (134) slotting into said recessed groove.

3. The bi-lumen breathing circuit septum assembly according to claim 1, **characterised by** the one end on which each of said first and second joints (11, 12) is connected to said circuit body being the connected end, and said connected end being inserted into the circuit body (131), with the partition plate (14) located within the cavity of the said connected end.

4. The bi-lumen breathing circuit septum assembly according to claim 3, **characterised by** said partition plate (14) partitioning the cavity of said connection end into two equal sections.

5. The bi-lumen breathing circuit septum assembly according to claim 3, **characterised by** a sealing groove (113) being provided on said connection end for the installation of sealing rings (112).

6. The bi-lumen breathing circuit septum assembly according to claim 5, **characterised by** the connecting point of said first joint (11) and said circuit body (131), and the connecting point of said second joint (12) and said circuit body (131) each being sealed and protected with an airtight plastic layer, with said airtight plastic layer being wrapped around the corresponding surfaces of said sealing rings (112).

7. The bi-lumen breathing circuit septum assembly according to any of claims 1-6, **characterised by** said circuit body being a corrugated pipe (134).

## Patentansprüche

1. Septumanordnung mit Bi-Lumen-Atmungskreis, umfassend: eine erste Verbindung (11) zum Verbinden einer Atemmaske und eine zweite Verbindung (12) zum Verbinden eines Beatmungsgerätes, wobei die erste Verbindung und die zweite Verbindung durch einen Bi-Lumen-Atmungskreis (13) verbunden sind, der ein zum Transportieren von Gasen verwendeter biegsamer Umlaufkörper (131) ist, in dessen Hohlraum ein Septum (134) enthalten ist, das den Bi-Lumen-Atmungskreislauf (13) in einen ersten und zweiten Kanal (132, 133) unterteilt, die gegenseitig isoliert sind,
weiter
umfassend: eine Trennwand (14) zum Unterteilen des Hohlraums der ersten Verbindung (11) in zwei voneinander isolierte Abschnitte; und eine Verbindungsstruktur (114), die an der Trennwand (14) für ihre Verbindung mit dem Septum (134) angeordnet ist, wobei ein Ende des Septums (134) mit der Verbindungsstruktur (114) verbunden ist; **dadurch gekennzeichnet, dass** die Anordnung weiter einen Testverschluss (2) umfasst, der in die erste Verbindung (11) einsetzbar ist, um entweder den ersten oder zweiten Kanal (132, 133) zu blockieren, um die Erfassung von Luftdichtheit des Septums des Bi-Lumen-Kreises zu erlauben.

2. Septumanordnung mit Bi-Lumen-Atmungskreis nach Anspruch 1, **dadurch gekennzeichnet, dass** die Verbindungsstruktur (114) eine vertiefte Nut ist, die auf der Trennwand (14) angeordnet ist, wobei das Septum (134) in die vertiefte Nut eingreift.

3. Septumanordnung mit Bi-Lumen-Atmungskreis nach Anspruch 1, **gekennzeichnet durch** das eine Ende, an dem jede der ersten und zweiten Verbindungen (11, 12) mit dem Umlaufkörper, der das verbundene Ende ist, verbunden ist und das verbundene Ende in den Umlaufkörper (131) eingesetzt ist, wobei die Trennwand (14) innerhalb des Hohlraums des verbundenen Endes angeordnet ist.

4. Septumanordnung mit Bi-Lumen-Atmungskreis nach Anspruch 3, **dadurch gekennzeichnet, dass** die Trennwand (14) den Hohlraum des Verbindungsendes in zwei gleiche Abschnitte unterteilt.

5. Septumanordnung mit Bi-Lumen-Atmungskreis nach Anspruch 3, **gekennzeichnet durch** eine an dem Verbindungsende vorgesehene Dichtungsnut (113) zum Einbau von Dichtungsringen (112).

6. Septumanordnung mit Bi-Lumen-Atmungskreis nach Anspruch 5, **gekennzeichnet durch** den Verbindungspunkt der ersten Verbindung (11) und des Schaltkreises (131) und den Verbindungspunkt der zweiten Verbindung (12) und des Schaltkreises (131), die jeweils mit einer luftdichten Kunststoffschicht abgedichtet und geschützt sind, wobei die luftdichte Kunststoffschicht um die entsprechenden Oberflächen der Dichtungsringe (112) gewickelt ist.

7. Septumanordnung mit Bi-Lumen-Atmungskreis nach einem der Ansprüche 1-6, **dadurch gekennzeichnet, dass** der Umlaufkörper ein Wellrohr (134) ist.

## Revendications

1. Ensemble de septum de circuit respiratoire à double lumière, comprenant : une première articulation (11) pour relier un masque respiratoire et une seconde articulation (12) pour relier un respirateur, avec ladite première articulation et ladite seconde articulation reliées par un circuit respiratoire à double lumière (13) qui est un corps de circuit flexible (131) utilisé pour transporter des gaz, à l'intérieur de la cavité duquel est contenu un septum (134) qui divise ledit circuit respiratoire à double lumière (13) en un premier et un second canal (132, 133) qui sont isolés l'un de l'autre, comprenant en outre : une plaque de séparation (14) pour séparer la cavité dudit premier joint (11) en deux sections isolées l'une de l'autre ; et une structure de liaison (114) située au niveau de la plaque de séparation (14) pour sa liaison avec ledit septum (134), avec une extrémité dudit septum (134) reliée à ladite structure de liaison (114) ; **caractérisé en ce que** l'ensemble comprend en outre un bouchon de test (2) pouvant être inséré dans ledit premier joint (11) pour bloquer soit le premier soit le second canal (132, 133) afin de permettre la détection de l'étanchéité du septum du circuit à double lumière.

2. Ensemble de septum de circuit respiratoire à double lumière selon la revendication 1, **caractérisé en ce que** ladite structure de liaison (114) est une gorge évidée située sur ladite plaque de séparation (14), avec ledit septum (134) s'insérant dans ladite gorge évidée.

3. Ensemble de septum de circuit respiratoire à double lumière selon la revendication 1, **caractérisé par** la première extrémité sur laquelle chacune desdites première et seconde articulations (11, 12) est reliée audit corps de circuit étant l'extrémité reliée, et ladite extrémité reliée étant insérée dans le corps de circuit (131), avec la plaque de séparation (14) située dans la cavité de ladite extrémité reliée.

4. Ensemble de septum de circuit respiratoire à double lumière selon la revendication 3, **caractérisé en ce que** ladite plaque de séparation (14) divise la cavité de ladite extrémité de liaison en deux sections égales.

5. Assemblage de septum de circuit respiratoire à double lumière selon la revendication 3, **caractérisé par** une rainure d'étanchéité (113) qui est ménagée sur ladite extrémité de liaison pour l'installation de bagues d'étanchéité (112).

6. Assemblage de septum de circuit respiratoire à double lumière selon la revendication 5, **caractérisé par** le point de liaison dudit premier joint (11) et dudit corps de circuit (131), et par le point de liaison dudit second joint (12) et dudit corps de circuit (131) qui sont chacun scellé et protégé par une couche de plastique étanche à l'air, ladite couche de plastique étanche à l'air étant enroulée autour des surfaces correspondantes desdites bagues d'étanchéité (112).

7. Assemblage de septum de circuit respiratoire à deux lumières selon l'une quelconque des revendications 1 à 6, **caractérisé en ce que** ledit corps de circuit est un tuyau ondulé (134).
